# EUROPEAN PATENT SPECIFICATION

(11) **EP 2 853 937 B1**
(45) Date of publication and mention of the grant of the patent: **01.11.2017**
(21) Application number: 13186191.6
(22) Date of filing: 26.09.2013
(51) Int. Cl.: G02C 5/00, G02C 11/00, A61F 9/02, A61B 3/11, A61B 5/00

(54) **Goggle-like mobile apparatus and method for recording images of a pupil of a person**
Goggle-ähnliche mobile Vorrichtung und Verfahren zum Aufnehmen von Bildern einer Pupille einer Person
Appareil mobile similaire à des lunettes et procédé permettant d'enregistrer des images d'une pupille d'une personne

(43) Date of publication of application: 01.04.2015
(73) Proprietor: Stickel, Manfred, 69257 Wiesenbach (DE)
(72) Inventor: Stickel, Manfred, 69257 Wiesenbach (DE)
(74) Representative: Witte, Weller & Partner Patentanwälte mbB

(56) References cited:
- WO-A1-86/04799
- WO-A1-2007/128034
- US-A1- 2005 099 601
- US-B1- 6 382 792

## Description

The present invention relates to a goggle-like mobile apparatus and a corresponding method for recording images of a pupil of a person. Still further, the present invention relates to a computer program for implementing said method.

Diabetes mellitus refers to a group of metabolic diseases causing high blood sugar values in a patient. Even if the number of patients suffering from the disease has been increasing for years, diabetes is nowadays considered comparatively treatable. Main sources for cost-intensive complications during treatment are associated illnesses accompanying the diabetes. One important associated illness is diabetic neuropathy, in particular diabetic autonomic neuropathy (DAN), which affects approximately half of all diabetes patients after 10 years of their first diabetes diagnosis. Autonomic neuropathy is an autonomic nerve disorder and may lead to multiple problems such as the loss of sensation in the extremities or even complete blindness in extreme cases. However, an early diagnosis of a beginning DAN allows adapting an on-going diabetes therapy and thereby reducing the effects of the long-term consequences. This again leads to relief for the patients but also to costs savings in the health system.

It is non-controversial that the early detection of DAN by means of a wide-spread screening of diabetes patients could help to save costs. However, it is difficult to implement such a screening efficiently considering the number of patients and the duration of a suitable examination by physicians or specialists.

One possible examination approach for detecting a beginning DAN is based on the measurement of the response of a patient's pupil to a light stimulus. The size of pupil adapted to darkness shows a characteristic behavior of contraction and expansion in response to illumination, e.g. in response to a light flash (pupillary light reflex or pupillary light response). A considerable amount of studies has been carried out since the 1960s in order to evaluate the effects of diabetes on the pupillary light reflex of patients. In particular, one result of these studies is that changes in the response of a pupil to a light stimulus are detectable prior to the appearance of clinical symptoms of a DAN. These effects can be measured by means of pupillometry.

US 2005/099601 A1 discloses a goggle based light-weight VOG system includes at least one digital camera connected to and powered by a laptop computer through a firewire connection. The digital camera may digitally center the pupil in both the X and Y directions. A calibration mechanism may be incorporated onto the goggle base. An EOG system may also be incorporated directly into the goggle. The VOG system may track and record 3-D movement of the eye, track pupil dilation, head position and goggle stoppage. An animated eye display provides data in a more meaningful fashion. The VOG system is a modular design whereby the same goggle frame or base is used to build a variety of digital camera VOG systems.

WO 2007/128034 A1 discloses a portable device in the form of a mask that can be worn over a user's eyes is provided with a non-visible light source to illuminate the user's eyes, and a lens arrangement and image capturing means to capture digital images of the eyes that can then be processed and used to diagnose a variety of dizziness and balance-related disorders and disease. The mask includes a light-omitting seal or cover to prevent external light reaching the eyes. The images can then be sent via data transmitter to an external device for processing and/or further analysis. The mask has particular suitability for use remotely by patients themselves during a dizziness episode.

In Zangemeister et al. "Pupillary responses to single and sinusoidal light stimuli in diabetic patients" (Neurology International 01/2009; 1:e19) the authors examine the effect of diabetes on the pupillary light reflex. 52 patients suffering from diabetes and 21 control subjects are evaluated and the authors try to answer the questions whether recording and frequency analysis of different parameters deduced from the pupillary light response to light stimuli allows differentiating between diabetes patients suffering from diabetes and other subjects. The authors conclude that significant changes in the pupillary light response in diabetes patients can be found only when symptoms of autonomic neuropathy have been shown. Although the study also evaluates differences between different approaches to evaluate the pupillary light response, the basic result is that the pupillary light response in combination with information processing shows significant differences if the patient not only suffers from diabetes but also shows symptoms of autonomic neuropathy.

One difficulty with this method is that the apparatuses to measure the pupillary light reflex are usually bulky, expensive and difficult to handle and thus mainly suitable for studies in controlled environments like hospitals. This makes the realization of a wide-spread screening procedure more difficult because of the required expert input during the examination.

It is an object of the present invention to overcome the above-mentioned problems. Particularly, it is an object of the present invention to provide an easy and cost-effective approach for deriving information on autonomic nerve or ocular disorders affecting the static and dynamic behavior of the human pupil, in particular a potential DAN, before chronic symptoms surface.

According to a first aspect of the present invention, there is provided a goggle-like mobile apparatus for recording images of a pupil of a person suitable for self-monitoring and deriving information on autonomic nerve disorders or ocular disorders of said person, said apparatus comprising a light-tight housing for covering both eyes of the person including a separation wall for light-tight sealing one eye from the other, illumination means for illuminating at least one eye of the person, stimulation means for stimulating at least one eye of the person with a light stimulus to trigger a pupillary light reflex and recording means for recording images of at least one eye at least during a predetermined time period following the stimulation of at least one eye. According to a further aspect of the present invention, there is provided a method for recording images with an apparatus as described above.

The proposed apparatus according to the first aspect of the present invention has the shape of a pair of goggles and is lightweight and mobile. By including the above-mentioned features in a goggle-like apparatus, images of an eye of a person can be recorded without previously requiring exact adjustment of the position of the apparatus

With such a user interface, the automated measurement of pupillary parameters becomes possible or is at least facilitated. If the user himself can initiate a recording, i.e. a measurement procedure, by means of a trigger signal it can be assured that the person is concentrated during the measurement. It may lead to unintended side effects if a person is surprised by the measurement procedure, in particular by a light stimulation. A user interface helps to solve this problem.

If it is further possible for the user to assure that the pupil orientation is adequate, the quality of the recorded images could be improved. It is only possible to perform an adequate data analysis or image processing if the pupil orientation is as much as possible directed to the camera. Further, if the pupils are not oriented towards the optical axis of the camera the focusing might become more difficult. By allowing the person to confirm that the eyes are oriented versus the orientation means and consequently appropriately focused in order to record suitable images for the data analysis or image processing, the number of recordings with invalid results can be reduced. The same reasoning also holds for incident light in the light-tight housing which could also worsen the quality of the recorded images. If feedback is obtained on the positioning of the goggle-like apparatus, especially the light-tight housing's orientation versus the person's face, errors resulting from a wrong adjustment can be reduced.

After recording and evaluating images, the user interface can be used to provide direct feedback to the user on the outcome of the measurement.

According to yet another aspect of the present invention there is provided a computer program comprising program code means for causing a computer to carry out the steps of the methods according to the present invention when said computer program is carried on the computer.

It is to be understood that the features mentioned above and those yet to be explained below can be used not only in the respective combinations indicated but also in other combinations or as isolated features, without leaving the scope of the present invention. Preferred embodiments of the invention are defined in the dependent claims. It shall be understood that the claimed methods and computer program have similar and/or identical preferred embodiments as the claimed apparatus and as defined in the dependent claims.
- Fig. 1: shows an illustration of a goggle-like mobile apparatus according to the present invention;
- Fig. 2: shows a schematic illustration of a first embodiment of the present invention;
- Fig. 3: shows a schematic illustration of another embodiment of the present invention;
- Fig. 4: shows a schematic illustration of yet another embodiment of the present invention;
- Fig. 5: shows a schematic illustration of yet another embodiment of the present invention;
- Fig. 6: shows a schematic illustration of various optional features;
- Fig. 7: shows an overview of a method according to another aspect of the present invention; and
- Fig. 8: shows a schematic illustration of an apparatus according to another aspect of the present invention.

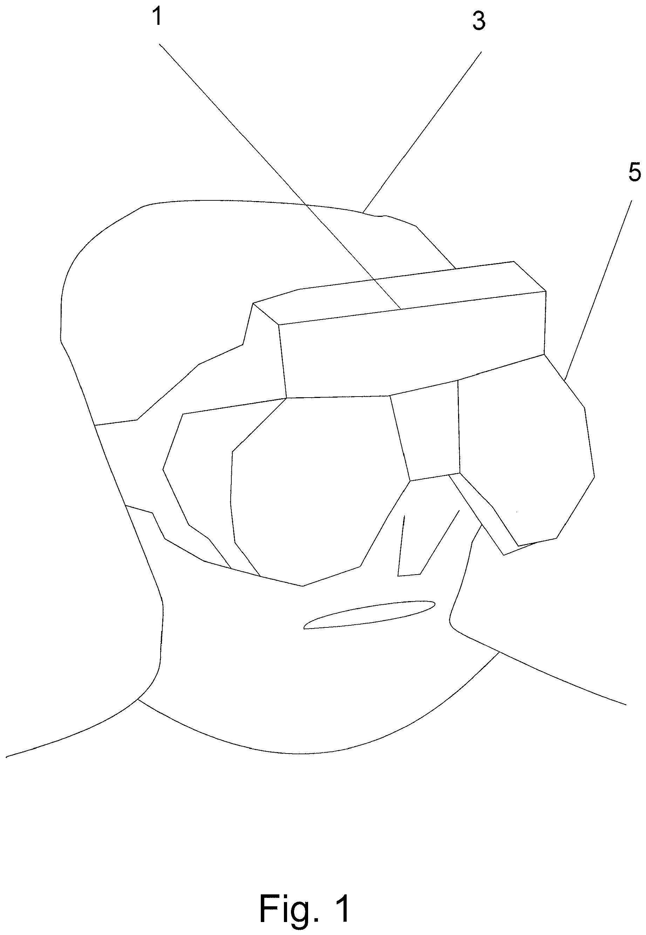

In Fig. 1 an illustration of an embodiment of a goggle-like mobile apparatus 1 according to the present invention is shown. A person 3 is able to cover both eyes in a light-tight housing 5 in order to record images of a pupil. One particular advantage of the apparatus according to the present invention is that it allows a self-monitoring (self-measurement), i.e. recording images of the eye of the person that is wearing the apparatus. Therefore, it is an important point in the design of the apparatus according to the present invention to provide an easy-to-manage and lightweight apparatus, which can be applied by a person himself for self-monitoring without consulting a physician or specialist. It is further important that no extensive system set-up procedure or determination of properties of a specific user is necessary.

By designing a measurement device in the form of a pair of goggles (goggle-like mobile apparatus) operation errors can be minimized as the position versus the eyes and pupils of the person wearing the apparatus is more or less fixed. One notable difference to current, state of the art, devices for measuring the pupillary light reflex is that the apparatus according to the present invention is smaller and portable. Possible designs of the present invention are comparable to welding goggles or diving goggles, possibly with an attached housing for a processor for the data processing.

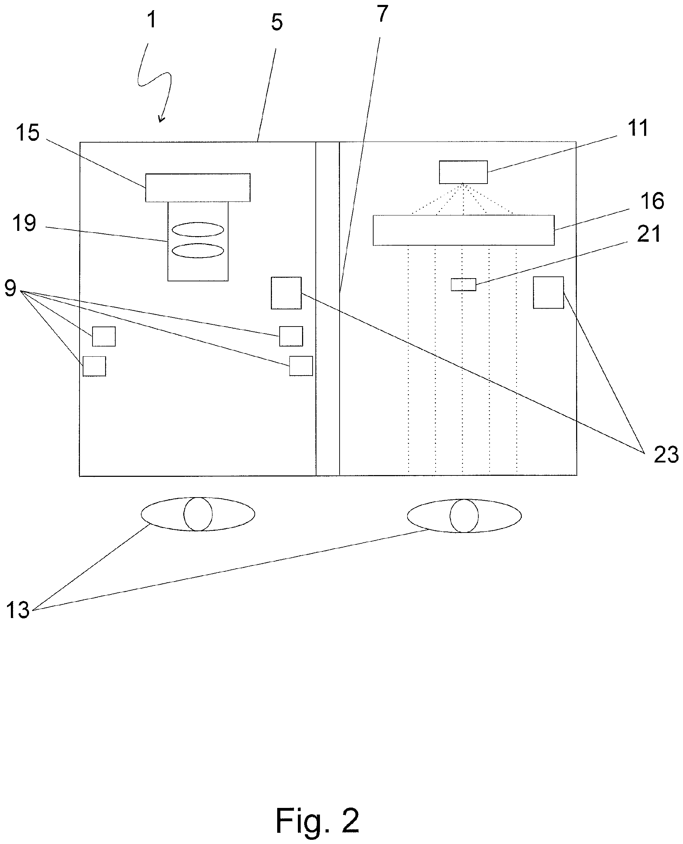

Fig. 2 shows a schematic illustration of an apparatus 1 according to one embodiment of the present invention. The light-tight housing 5 covers both eyes of a person and includes a separation wall 7 for light-tight sealing one eye from the other.

It is an important aspect of the present invention to allow undisturbed recording under controlled environmental conditions. The light-tight housing 5 shields both eyes from any external incident light. The separation wall 7 assures that one eye can be stimulated without disturbing a possible recording of the other eye on the other side of the separation wall. Eye movements and pupil reactions to external stimuli in both eyes of a person are connected physiologically. Thus, if one pupil is stimulated, e.g. by means of a light flash, not only this eye but also the other eye reacts with a contraction. The reaction of the other, i.e. contralateral, pupil is usually equivalent to the reaction of the originally stimulated pupil (consensual reaction). According to further embodiments of the present invention it is also possible to simultaneously stimulate and record images of both eyes of the person

The apparatus 1 further comprises illumination means 9 for illuminating at least one eye of the person. The measurement of the pupillary light reflex of the contralateral eye can be conducted more efficiently if there is no visible light causing unpredictable adaption reactions of the eye-to-be-measured. However, it is not possible to process images recorded in total darkness. Thus, illumination means 9 are used to illuminate at least one eye of the person, preferably in a predefined and reproducible way.

Further, the apparatus 1 comprises stimulation means 11 for stimulating at least one eye 13 of the person to trigger a pupillary light reflex. This stimulation means 11 can particularly be designed in the form of a light stimulus, e.g. a flashlight or an LED allowing sequentially altering the illumination level according to a predefined sequence. One possibility is that the stimulation means comprise at least one LED for emitting a flash in the visual light spectrum. It is also possible to configure the stimulation means 11 to illuminate at least one eye for a predefined time to a predefined level. Like this, different sequences of light and dark periods can be generated and provoke reactions of the pupils of the person under test. These reactions can e.g. be a pupillary light reflex, which refers to the reflex, i.e. reaction, to sudden illumination as well as to the reflex to sudden darkness (also pupillary dark reflex). Depending on the exact definition of the predefined time or level at which the LED 15 is illuminated different reactions (e.g. varying intensity or magnitude) might be triggered.

In order to provide a homogeneous light a generated flash might be diffused with diffusing means 17, in particular a diffusor. This diffusor 17 expands the generated light flash. Usually a white LED 11 is observed by the eye (the pupil) as a point source in spite of the small distance to the eye. The illumination level on the retina in the pupil might thus already decrease during the light flash due to the decreasing diameter of the pupil. In order to avoid a bias on the measurement results, the diffusor 17 produces a seemingly expanded and homogenized light source for the eye which may compensate of some part of the above-mentioned effect of the decreasing retina illumination with decreasing pupil size during the light flash.

Still further, recording means 15 are provided for recording images of at least one eye at least during a predetermined time period following the stimulation of at least one eye 13. One particular embodiment of the recording means 15 comprises a camera being sensitive in the infrared or near-infrared spectrum. If the illumination means 9 include at least one infrared or near-infrared LED, the recording means, i.e. the camera, allow recording undisturbed images in the infrared or near-infrared spectrum. Because the eyes are illuminated in a non- or barely visible wavelength, the reactions of the pupil to the stimulus are not compromised.

The camera 15 may also comprise focus means 19 which allow focusing on one eye 13 of the person. Differently shaped faces could result in different positions of the pupil versus the camera 15. In order to generate usable images, the camera 15 needs to compensate for different distances to the eye 13. Possible focus means 19 can thereby be lens-constructions that are manually (e.g. by means of set-screws) or electronically (e.g. by means of a motor-actuator) adjustable, (fixed) lenses with variable thickness or others. Further, the focus means 19 may be implemented in form of an electronically focusable objective transparent to the wavelength of the illumination means. Then, the adaption of the focus can be implemented in software and an autofocus (autofocus means) may be realized by including the focusing means in an automated control loop. Also, a definition of the reproduction scale to convert the measured pupil size from pixels into an appropriate dimension, e.g. millimeters, can be determined from such electronic focus means.

In order to cause a deterministic pupil orientation before triggering a recording there may also be provided optical orientation means 21 which form a visual reference point for at least one eye of the person. In particular, at least one LED with adjustable intensity can fulfill this function. These optical orientation means 21 may preferably be mounted close to the optical axis between the pupil 13 and the recording means 15. Due to the consensual movement of both pupils it is possible to mount the optical orientation means, i.e. the LED, 21 on one side of the separation wall 7 and cause the pupil on the other side of the separation wall 7 to be oriented in the same direction, i.e. to be directly oriented in the direction of the recording means 15. In complete darkness the lack of a reference point can cause unwilling movements of the eyes which may result in orientations away from the optical axis to the camera 15. This again can result in deformations of the circular-shaped pupil in the recorded images, which would require an algorithmic compensation approach. To cope with this, optical orientation means 21 can be realized in form of a point-shaped fixation light source emitting a light with intensity marginally over the visible threshold for providing a visual anchor to the pupil and reduce eye movements. Alternatively (not illustrated), the optical orientation means 21 may also be realized in form of two separate light sources arranged symmetrically versus the optical axis so that a user may orient his pupil through them. This allows providing horizontal as well as vertical alignment for the pupils. The recorded images thus usually show a circle-shaped pupil, which is well-suited for the image processing and data analysis.

The apparatus further comprises monitoring means 23, for monitoring the illumination within the light-tight housing 5 on at least one side of the separation wall 7. In particular, these monitoring means 23 can be in the form of a photodetector, e.g. a photodiode or photosensitive element, which allows converting the illumination level or light level in at least one side of the light-tight housing 5 into a digital or analog value for further processing. The main scope of the monitoring means is to control whether the goggle-like mobile apparatus 1 is applied correctly, i.e. no external light disturbs the measurement by influencing one of the pupils 13 of the person. For this, the background illumination is monitored on at least one side of the separation wall 7. A too high level of background illumination would influence the size of the pupil adapted to darkness or to a certain level of illumination and thus compromise the validity of the measurement results.

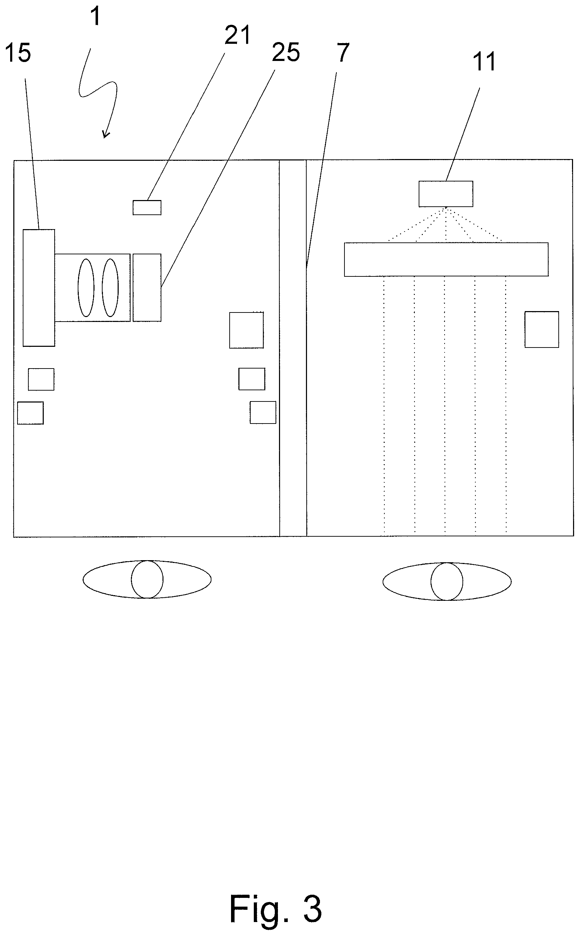

In Fig. 3 a schematic illustration of another embodiment of an apparatus 1 according to the present invention is presented. In comparison to the first embodiment, additionally light partition means 25 are comprised. These light partition means 25 are used for coupling the recording means 15, i.e. the camera, and the optical orientation means 21 into one optical path to an eye of the person. This embodiment thus allows combining camera 15 and optical orientation means 21 on the same side of the separation wall 7, i.e. simultaneously record and provide orientation to the same pupil. It is one advantage of this construction that the eye is ideally oriented versus the recording means 15 and that the stimulation means 11 on the other side of the separation wall 7 might be constructed in a more efficient way. In particular, these light partition means 25 can be in form of a mirror, e.g. a one-way mirror, or a prism. According to further embodiments of the present invention (not illustrated), the light partition means may also couple the illumination means and/or the stimulation means into one optical path. This may allow other mechanical designs and provide further advantages such as, e.g., a smaller or lighter housing.

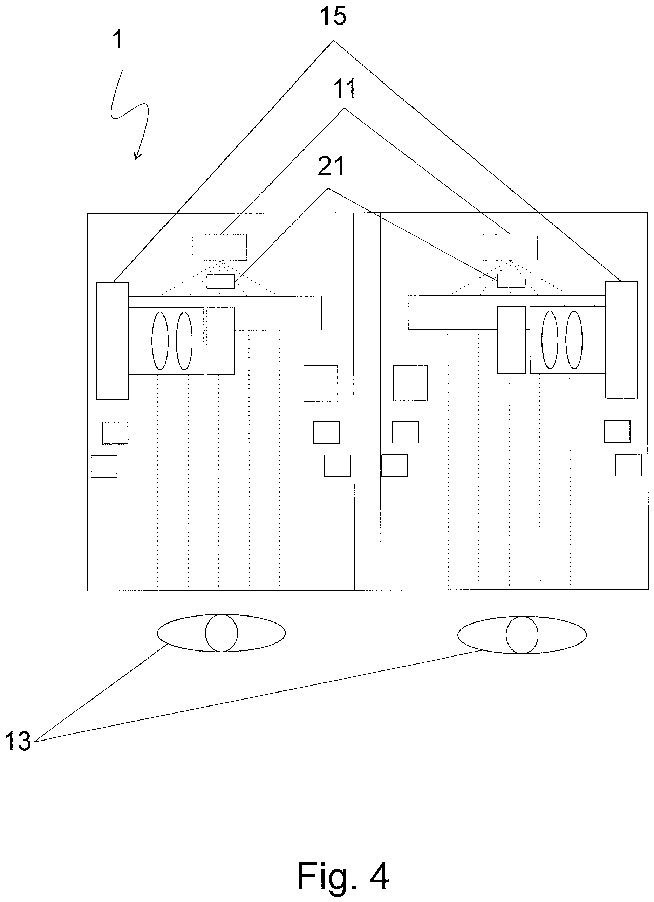

In Fig. 4 again another embodiment of the apparatus 1 according to the present invention is illustrated. In this embodiment both eyes of the person can be stimulated and images of both pupils 13 can be recorded. By providing stimulation means 11, optical orientation means 21 and recording means 15 on both sides of the separation wall 7 this effect can be achieved. In particular, one advantage of such a construction may be that variances of the pupillary light reflex of the two eyes 13 of the same person may be compensated. This construction thus allows a higher flexibility in the set-up phase for a measurement procedure, e.g. in the sequence of light and dark periods for the stimulation of a pupillary light reflex. Further, this design allows simultaneously stimulating and recording images of the same eye or of each eye individually. Still further, a redundant design allows to flexibly react to failures and inaccuracies caused by single parts.

Also, additional information can be obtained if it is possibly to simultaneously or subsequently record images of both eyes of a person and determine pupillary parameters therefrom. In particular, possible asymmetries in the reactions or a disorder of the consensual reaction of the two pupils/eyes can be discovered, evaluated and/or compensated. If, e.g., both eyes are simultaneously subject to stimulation and recording, a different behavior could be evaluated in order to determine whether the nerves connecting one of the eyes with the brain might be disturbed. If, e.g., one eye is illuminated for a certain time period (possibly up to several minutes) to a specified illumination level followed by a darkness period and an illumination of the other eye, then recording both eyes might allow evaluating how the dark-adaption sizes of the pupils and their reaction rates differ. The same is possible in various other forms also for stimulation with a visible light flash.

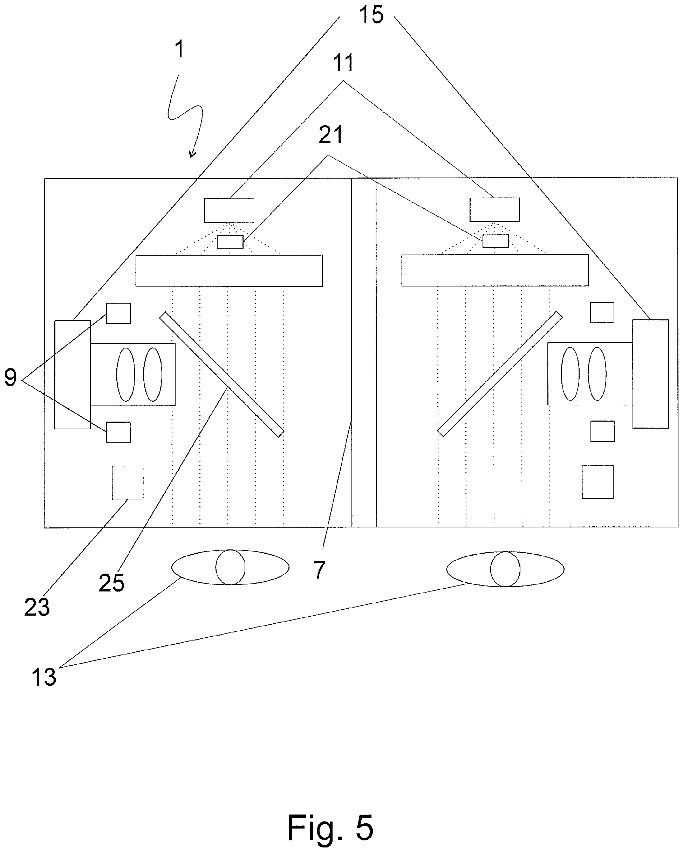

In Fig. 5 an alternative embodiment of the apparatus is illustrated schematically in order to show another possible positioning of the different components. The apparatus 1 again comprises stimulation means 11, optical orientation means 21 and recording means 15 on both sides of the separation wall 7. In this illustration, however, the light partition means 25 are larger in comparison to the previous embodiment. This might be necessary due to space requirements of the cameras 15 or a mirror or prism representing the light partition means 25 and being only producible in a larger size. In this embodiment, a mirror is used, which is transparent for a large part of the visible light spectrum but reflects light in the infrared or near-infrared spectrum. The illumination means 9, i.e. the infrared or near-infrared LEDs could then be arranged close to the recording means 15 in a position, which allows the generated illumination light to be reflected in the direction of the eyes 13 of the person. Further, the monitoring means 23 might be positioned in an alternative position as illustrated. Although not illustrated, different other (relative) positions of the components may lead to other advantages and lie within the scope of the present invention.

In further alternative embodiments, a mirror representing the light partition means might be oriented the other way around. Also the exact positioning of all elements within the light-tight housing might be varied. Depending on the shape of the other components, it could, e.g., be necessary to place the monitoring means at another position in order to allow an appropriate positioning of, e.g. the recording means versus the light partition means.

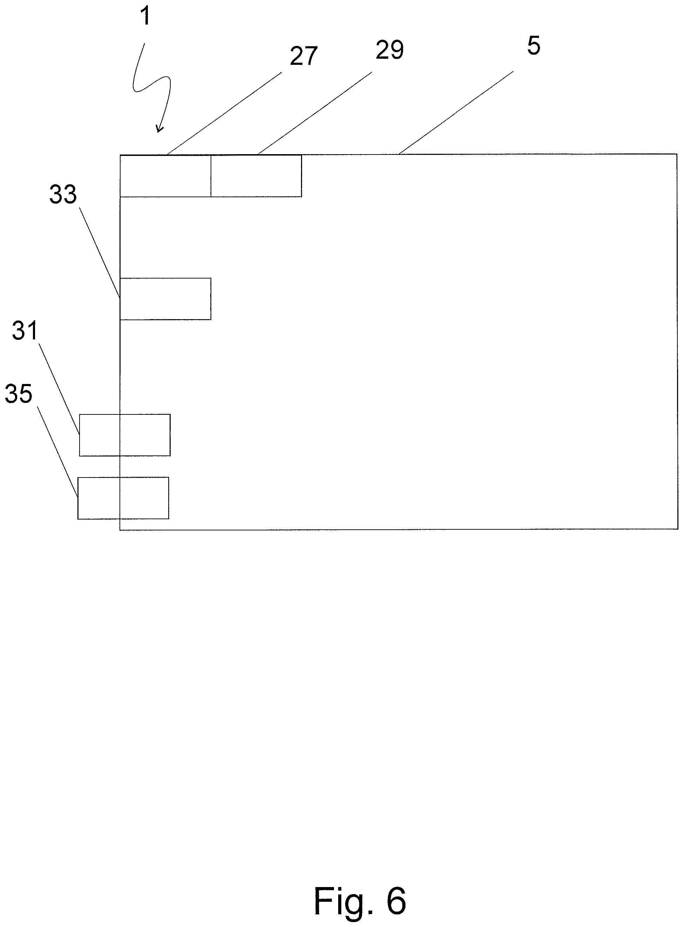

In Fig. 6 a goggle-like mobile apparatus is shown that additionally comprises data analysis means 27 for extracting the pupil size and its change over time from the recorded images and for determining at least one pupillary parameter characterizing the pupillary light reflex. Further comprised are evaluation means 29 for comparing the at least one pupillary parameter to previously determined pupillary parameters for the same person and for determining therefrom whether a predetermined criterion is fulfilled.

Most notably such a predetermined criterion can be a threshold based comparison of the time it takes for the pupil to contract after a light stimulus. Based on an image-processing (data analysis) extraction of the pupil size in each frame of the recorded images it is possible to calculate the size of the pupil in each image. From this, it can be determined how long it takes for the pupil to reach the maximum contraction. Other pupillary parameters might also be related to the size of the pupil at the beginning and after the stimulation, at the size difference at different illumination levels or at the time it takes for the pupil to adapt to a certain illumination level or others. In this context, the pupillary light reflex refers to the reaction of the pupil of a person to a change in the illumination level. This change in illumination level can for example be a flashlight or a slower change of illumination or also a change from light to darkness, which is also sometimes referred to as pupillary dark reflex. Then, based on these determined parameters, it is checked whether a predetermined criterion is fulfilled. Apart from simple thresholding, time series analysis methods can be applied.

Further, in Fig. 6, a data interface 31 for transferring recorded images, pupillary parameters and/or control information between the apparatus and an external device is shown. This data interface 31 can, e.g., be a universal serial bus (USB) or a parallel interface allowing a higher data transmission rate. Also possible is a wireless interface allowing to wirelessly transmit data to the goggle-like mobile apparatus 1 and/or to wirelessly obtain data from the apparatus. In case of a cable-based interface, different plugs and protocols are possible for this data interface 31. One main goal of this data interface 31 is to provide a possibility for the user to store and/or further process (image processing, data analysis) the recorded data on another device, e.g. a personal computer, smartphone or tablet PC. It might also be advantageous to combine a cable-based data interface with an electrical power interface for charging an accumulator also optionally comprised in the apparatus 1.

It is further possible to also include storage means 33 for storing recorded images and/or pupillary parameters determined from recorded images. Such storage means may, e.g., be realized in form of an SD-card or another type of flash memory. One objective of these storage means 33 is to allow the recording of images and the determination of pupillary parameters therefrom for a longer time period. This time period may be several days, weeks, or months. By, e.g., storing information over several weeks of a pupillary parameter, e.g. the time it takes for a pupil to reach a certain fraction of its original size after stimulation, a long-term tendency can be observed. For this long-term tendency it is also possible to apply statistical techniques, e.g. time series analysis, in order to determine statistically significant results about the progress of an illness, e.g. DAN. In fact, all illnesses causing autonomic nerve or ocular disorders and/or affecting the static and dynamic behavior of the human pupil might be detected.

Still further, the apparatus might additionally comprise a user interface 35 allowing the person using the apparatus to initiate a recording by providing an external trigger signal, confirm that the optical orientation means 21 are visible and thus that the camera orientation is adequate, obtain feedback on incident light in the light-tight housing 5 and a possibly resulting need for adjusting the position of the apparatus 1 versus the person's face 3 and/or obtain feedback whether at least one determined pupillary parameter fulfills a predetermined criterion. This user interface 35 may provide visual information, e.g. by means of a small display or acoustic information, different sounds for different procedures or even haptic feedback, e.g. vibration in case a certain step in the measurement has been accomplished successfully. Other possibilities for feedback might include differently colored LEDs, spoken directions from a recorded voice or others. The main scope of providing feedback to the person wearing the mobile apparatus is to assure that the positioning of the apparatus, in particular of the camera, versus the person's face is adequate.

The user feedback can be entered by means of buttons, a touchscreen interface, speech processing or others, e.g., in order to provide further information or to start a measurement. According to one specific embodiment the main purpose of the feedback is to provide information to the users if the apparatus needs better adjustment and/or to provide information on the outcome of the measurement as soon as the measurement is finished. Further, it is important to provide the user with information on the outcome of the measurement. In particular, feedback is desired if a certain pupillary parameter, as determined by the evaluation means 29 fulfills a predetermined criterion. The user might take this feedback as an indication for him to visit a doctor or specialist.

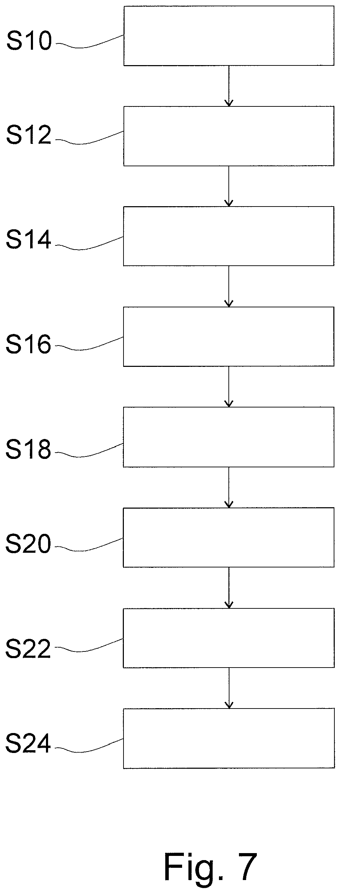

In Fig. 7 a method for recording images of a pupil of a person suitable for self-monitoring and deriving information on autonomic nerve disorders or ocular disorders of said person according to one aspect of the present invention is outlined. At first, both eyes of the person are covered light-tight and sealed from one another S10. Then, at least one eye of the person is illuminated with illumination means S12. After this, at least one eye of the person is stimulated and a pupillary light reflex is triggered S14. This stimulation can be in form of a light flash in visible light spectrum or also in the form of an increase/decrease of the illumination level over a certain time. It is also possible to suddenly switch-off of an LED. Next, recording means are used to record images of at least one eye at least during a predetermined time period following the stimulation of one eye S16.

Optionally, a method according to the present invention might also comprise further steps in order to derive information on any autonomic nerve or ocular disorder affecting the static and dynamic behavior of the human pupil. One example for such a disorder can be a DAN. In order to determine these data the pupil size is extracted S18 with particular regard to its change over time. Based on these images, at least one pupillary parameter characterizing the pupillary light reflex is determined S20. This pupillary parameter is compared S22 to previously determined pupillary parameters for the same person. The comparison to parameters for the same person, i.e. a self-reference, avoids the spread in the reactions of the pupils of a group of persons such that parameter changes can be identified faster. Based on this at least one pupillary parameter, it is determined S24 whether a predetermined criterion is fulfilled as outlined above.

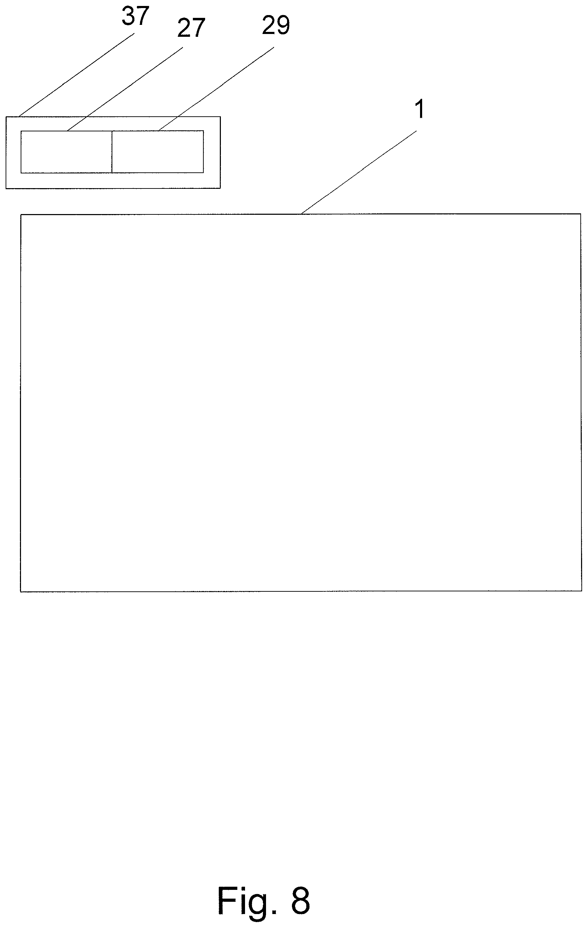

In Fig. 8, yet another aspect of the present invention is shown. According to this embodiment separate data analysis means 27 and evaluation means 29 process data obtained from an apparatus 1 allowing the recording of images of a pupil of a person. Such separate data analysis means 27 and evaluation means 29 may, e.g., be designed as a separate apparatus 37 or may also be incorporated in a personal information processing device like a smartphone or the like, or also in a computer.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program or computer-implemented method may be stored/distributed on a suitable non-transitory medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. Goggle-like mobile apparatus (1) for recording images of a pupil of a person (3) suitable for self-monitoring and deriving information on autonomic nerve disorders or ocular disorders of said person, said apparatus comprising
- illumination means (9) for illuminating at least one eye of the person (3);
- stimulation means (11) for stimulating at least one eye of the person (3) with a light stimulus to trigger a pupillary light reflex; and
- recording means (15) for recording images of at least one eye at least during a predetermined time period following the stimulation of at least one eye,
**characterized by** a light-tight housing (5) for covering both eyes of the person (3) including a separation wall (7) for light-tight sealing one eye from the other.

2. Apparatus (1) according to claim 1, further comprising optical orientation means (21), in particular at least one LED with adjustable intensity, within the light-tight housing (5) for providing a visual reference point for at least one eye of the person.

3. Apparatus (1) according to claim 2, further comprising light partition means (25), in particular a mirror or a prism, for coupling the illumination means (9), the stimulation means (11), the recording means (15) and/or the optical orientation means (21) into one optical path to an eye of the person (3).

4. Apparatus (1) according to any one of the preceding claims, wherein
- the illumination means (9) are configured to illuminate the first eye of the person (3);
- the stimulation means (11) are configured to stimulate the second eye of the person (3); and
- the recording means (15) are configured to record images of the first eye of the person (3) at least during a predetermined time period following the stimulation of the second eye.

5. Apparatus (1) according to any one of the preceding claims, further comprising monitoring means (23), in particular at least one photodetector, for monitoring the illumination within the light-tight housing (5) on at least one side of the separation wall (7).

6. Apparatus (1) according to any one of the preceding claims, wherein
- the illumination means (9) comprise at least one infrared or near-infrared LED for providing uniform illumination of at least one eye of the person (3); and/or
- the stimulation means (11) comprise at least one LED for emitting a flash in the visual light spectrum and/or for illuminating at least one eye of the person (3) for a predefined time to a predefined level.

7. Apparatus (1) according to claim 6, further comprising light diffusing means (17) for homogenizing and enlarging the apparent visual appearance of the generated flash.

8. Apparatus (1) according to any one of the preceding claims, wherein the recording means (15) comprise a camera being sensitive in the infrared or near-infrared spectrum, in particular a camera including focus means (19) for focusing on one eye of the person (3).

9. Apparatus (1) according to any one of the preceding claims, further comprising
- data analysis means (27) for extracting the pupil size and its change over time from the recorded images and for determining at least one pupillary parameter characterizing the pupillary light reflex; and
- evaluation means (29) for comparing the at least one pupillary parameter to previously determined pupillary parameters for the same person (3) and for determining therefrom whether a predetermined criterion is fulfilled.

10. Apparatus (1) according to any one of the preceding claims, further comprising
- a data interface (31) for transferring recorded images, pupillary parameters and/or control information between the apparatus (1) and an external device; and/or
- storage means for storing recorded images and/or pupillary parameters determined from recorded images.

11. Apparatus (1) according to any one of the preceding claims, further comprising a user interface (35) allowing the person (3) to
- initiate a recording by providing an external trigger signal;
- confirm that the optical orientation means (21) are visible and thus that the camera orientation is adequate;
- obtain feedback on incident light in the light-tight housing (5) and a possibly resulting need for adjusting the position of the apparatus (1) versus the person's (3) face; and/or
- obtain feedback whether at least one determined pupillary parameter fulfills a predetermined criterion.

12. Method for recording images of a pupil of a person (3) suitable for self-monitoring and deriving information on autonomic nerve disorders or ocular disorders of said person, said method comprising
- illuminating (S12) at least one eye of the person with illumination means;
- stimulating (S14) at least one eye of the person with a light stimulus to trigger a pupillary light reflex; and
- recording (S16) images of at least one eye at least during a predetermined time period following the stimulation of one eye,
**characterized by** light-tight covering both eyes of the person and light-tight sealing (S10) one eye from the other with a light-tight housing (5) including a separation wall (7) for light-tight sealing one eye from the other.

## Patentansprüche

1. Brillen-ähnliche mobile Vorrichtung (1) zum Aufnehmen von Bildern einer Pupille einer Person (3), die zur Selbstüberwachung und zur Ableitung von Informationen über autonome Nervenstörungen oder Augenerkrankungen der Person geeignet ist, wobei die Vorrichtung umfasst:
- Beleuchtungsmittel (9) zum Beleuchten mindestens eines Auges der Person (3);
- Stimulationsmittel (11) zum Stimulieren von mindestens einem Auge der Person (3) mit einem Lichtreiz, um einen Pupillenlichtreflex auszulösen; und
- Aufzeichnungsmittel (15) zum Aufzeichnen von Bildern von mindestens einem Auge zumindest während einer vorgegebenen Zeitspanne nach der Stimulation von mindestens einem Auge,
**gekennzeichnet durch** ein lichtdichtes Gehäuse (5) zum Abdecken beider Augen der Person (3) mit einer Trennwand (7) zur lichtdichten Abschottung eines Auges vom anderen.

2. Vorrichtung (1) nach Anspruch 1, ferner mit optischen Orientierungsmitteln (21), insbesondere mindestens einer LED mit einstellbarer Intensität, innerhalb des lichtdichten Gehäuses (5) zur Bereitstellung eines visuellen Referenzpunktes für mindestens ein Auge der Person.

3. Vorrichtung (1) nach Anspruch 2, ferner mit Lichttrennmitteln (25), insbesondere einem Spiegel oder einem Prisma, um die Beleuchtungsmittel (9), die Stimulationsmittel (11), die Aufzeichnungsmittel (15) und/oder der optischen Orientierungsmittel (21) in einen optischen Pfad zu einem Auge der Person (3) zu koppeln.

4. Vorrichtung (1) nach einem der vorherigen Ansprüche, wobei
- die Beleuchtungsmittel (9) so ausgestaltet sind, dass sie das erste Auge der Person (3) beleuchten;
- die Stimulationsmittel (11) so ausgestaltet sind, dass sie das zweite Auge der Person (3) stimulieren; und
- die Aufzeichnungsmittel (15) so ausgestaltet sind, dass sie Bilder des ersten Auges der Person (3) zumindest während einer vorgegebenen Zeitspanne nach der Stimulation des zweiten Auges aufzeichnen.

5. Vorrichtung (1) nach einem der vorherigen Ansprüche, ferner mit Überwachungsmitteln (23), insbesondere mindestens einem Photodetektor, zur Überwachung der Beleuchtung innerhalb des lichtdichten Gehäuses (5) auf mindestens einer Seite der Trennwand (7).

6. Vorrichtung (1) nach einem der vorherigen Ansprüche, wobei
- die Beleuchtungsmittel (9) mindestens eine Infrarot- oder Nah-Infrarot-LED aufweisen, um eine gleichmäßige Ausleuchtung von mindestens einem Auge der Person (3) zu bieten; und/oder
- die Stimulationsmittel (11) mindestens eine LED zum Aussenden eines Blitzes im visuellen Lichtspektrum und/oder zum Beleuchten mindestens eines Auges der Person (3) für eine vorgegebene Zeit auf einem vorgegebenen Level aufweisen.

7. Vorrichtung (1) nach Anspruch 6, ferner mit Lichtstreuungsmitteln (17) zum Homogenisieren und Vergrößern des scheinbaren visuellen Erscheinungsbildes des erzeugten Blitzes.

8. Vorrichtung (1) nach einem der vorherigen Ansprüche, wobei die Aufzeichnungsmittel (15) eine Kamera umfassen, die im Infrarot- oder Nah-Infrarot-Spektrum empfindlich ist, insbesondere eine Kamera mit Fokuseinrichtung (19) zum Fokussieren auf ein Auge der Person (3).

9. Vorrichtung (1) nach einem der vorherigen Ansprüche, ferner mit
- Datenanalysemitteln (27) zum Extrahieren der Pupillengröße und deren zeitlicher Änderung aus den aufgezeichneten Bildern und zum Bestimmen mindestens eines Pupillenparameters, der den Pupillenlichtreflex kennzeichnet; und
- Auswertungsmitteln (29) zum Vergleich des mindestens einen Pupillenparameters mit zuvor bestimmten Pupillenparametern für dieselbe Person (3) und zur Bestimmung daraus, ob ein vorgegebenes Kriterium erfüllt ist.

10. Vorrichtung (1) nach einem der vorherigen Ansprüche, ferner mit
- einer Datenschnittstelle (31) zum Übertragen von aufgezeichneten Bildern, Pupillenparametern und/oder Steuerinformationen zwischen der Vorrichtung (1) und einer externen Vorrichtung; und/oder
- Speichermitteln zum Speichern von aufgezeichneten Bildern und/oder Pupillenparametern, die aus aufgezeichneten Bildern ermittelt wurden.

11. Vorrichtung (1) nach einem der vorherigen Ansprüche, ferner mit einer Benutzerschnittstelle (35), die der Person (3) erlaubt,
- eine Aufzeichnung durch Bereitstellen eines externen Auslösesignals einzuleiten;
- zu bestätigen, dass die optischen Orientierungsmittel (21) sichtbar sind und somit die Kameraorientierung angemessen ist;
- Rückmeldung über einfallendes Licht in das lichtdichte Gehäuse (5) und eine sich eventuell ergebende Notwendigkeit, die Position der Vorrichtung (1) gegenüber dem Gesicht der Person (3) anzupassen, zu erhalten; und/oder
- Rückmeldung, ob mindestens ein bestimmter Pupillenparameter ein vorgegebenes Kriterium erfüllt, zu erhalten.

12. Verfahren zum Aufzeichnen von Bildern einer Pupille einer Person (3), das zur Selbstüberwachung und zur Ableitung von Informationen über autonome Nervenstörungen oder Augenerkrankungen dieser Person geeignet ist, wobei das Verfahren umfasst:
- Beleuchten (S12) mindestens eines Auges der Person mit Beleuchtungsmitteln;
- Stimulieren (S14) mindestens eines Auges der Person mit einem Lichtreiz, um einen Pupillenlichtreflex auszulösen; und
- Aufzeichnen (S16) von Bildern von mindestens einem Auge zumindest während einer vorgegebenen Zeitspanne nach der Stimulation eines Auges,
**gekennzeichnet durch** lichtdichtes Abdecken beider Augen der Person und lichtdichtes Abschotten (S10) eines Auges vom anderen mit einem lichtdichten Gehäuse (5) mit einer Trennwand (7) zur lichtdichten Abschottung eines Auges vom anderen.

## Revendications

1. Appareil mobile de type lunettes (1) pour enregistrer des images de la pupille d'une personne (3) permettant d'effectuer un contrôle automatique et de dériver des informations sur des troubles nerveux autonomes ou des troubles oculaires de ladite personne, ledit appareil comprenant :
- des moyens d'éclairage (9) pour éclairer au moins un oeil de la personne (3);
- des moyens de stimulation (11) pour stimuler au moins un oeil de la personne (3) avec un stimulus lumineux afin de déclencher un réflexe lumineux pupillaire ; et
- des moyens d'enregistrement (15) pour enregistrer des images d'au moins un oeil au moins pendant une période prédéterminée suivant la stimulation d'au moins un oeil ; **caractérisé par** un boîtier étanche à la lumière (5) pour recouvrir les deux yeux de la personne (3) comprenant une paroi de séparation (7) isolant un oeil par rapport à l'autre de manière étanche à la lumière.

2. Appareil (1) selon la revendication 1, comprenant en outre des moyens d'orientation optique (21), notamment au moins une LED avec une intensité ajustable, dans le boîtier étanche à la lumière (5) pour fournir un point de référence visuel pour au moins un oeil de la personne.

3. Appareil (1) selon la revendication 2, comprenant en outre un moyen de division de lumière (25), en particulier un miroir ou un prisme, pour coupler les moyens d'éclairage (9), les moyens de stimulation (11), les moyens d'enregistrement (15) et/ou les moyens d'orientation optique (21) dans un trajet optique vers un oeil de la personne (3).

4. Appareil (1) selon l'une quelconque des revendications précédentes, dans lequel :
- les moyens d'éclairage (9) sont conçus pour éclairer le premier oeil de la personne (3) ;
- les moyens de stimulation (11) sont conçus pour stimuler le second oeil de la personne (3) ; et
- les moyens d'enregistrement (15) sont conçus pour enregistrer des images du premier oeil de la personne (3) au moins pendant une période prédéterminée suivant la stimulation du second oeil.

5. Appareil (1) selon l'une quelconque des revendications précédentes, comprenant en outre un moyen de contrôle (23), notamment au moins un photo-détecteur, pour contrôler l'éclairage dans le boîtier étanche à la lumière (5) sur au moins un côté de la paroi de séparation (7).

6. Appareil (1) selon l'une quelconque des revendications précédentes, dans lequel :
- les moyens d'éclairage (9) comprennent au moins une LED infrarouge ou proche de l'infrarouge pour fournir un éclairage uniforme d'au moins un oeil de la personne (3) ; et/ou
- les moyens de stimulation (11) comprennent au moins une LED pour émettre un flash dans le spectre de la lumière visible et/ou pour éclairer au moins un oeil de la personne (3) pendant un temps prédéfini à un niveau prédéfini.

7. Appareil (1) selon la revendication 6, comprennent en outre un moyen de diffusion de lumière (17) pour homogénéiser et agrandir l'aspect visuel apparent du flash généré.

8. Appareil (1) selon l'une quelconque des revendications précédentes, dans lequel les moyens d'enregistrement (15) comprennent une caméra sensible dans le spectre infrarouge ou proche de l'infrarouge, notamment une caméra comprenant un moyen de focalisation (19) pour la focalisation sur un oeil de la personne (3).

9. Appareil (1) selon l'une quelconque des revendications précédentes, comprenant en outre :
- un moyen d'analyse de données (27) pour extraire la taille de la pupille et son changement dans le temps à partir des images enregistrées, et pour déterminer au moins un paramètre pupillaire caractérisant le réflexe lumineux pupillaire ; et
- un moyen d'évaluation (29) pour comparer l'au moins un paramètre pupillaire à des paramètres pupillaires préalablement déterminés for la même personne (3) et pour déterminer à partir de ceux-ci si un critère prédéterminé est rempli.

10. Appareil (1) selon l'une quelconque des revendications précédentes, comprenant en outre :
- une interface de données (31) pour transférer des images enregistrées, des paramètres pupillaires et/ou des informations de commande entre l'appareil (1) et un dispositif externe ; et/ou
- un moyen de stockage pour stocker des images enregistrées et/ou des paramètres pupillaires déterminés à partir d'images enregistrées.

11. Appareil (1) selon l'une quelconque des revendications précédentes, comprenant en outre une interface d'utilisateur (35) permettant à la personne (3) de :
- lancer un enregistrement en fournissant un signal de déclenchement externe ;
- confirmer que les moyen d'orientation optique (21) sont visibles et que l'orientation de la caméra est donc correcte ;
- obtenir un retour sur la lumière incidente dans le boîtier étanche à la lumière (5) et un besoin potentiellement résultant d'ajustement de la position de l'appareil (1) par rapport au visage de la personne (3) ; et/ou
- obtenir un retour indiquant si au moins un paramètre pupillaire déterminé remplit un critère prédéterminé.

12. Procédé d'enregistrement d'images de la pupille d'une personne (3) permettant d'effectuer un contrôle automatique et de dériver des informations sur des troubles nerveux autonomes ou des troubles oculaires de ladite personne, lequel procédé consiste à :
- éclairer (S12) au moins un oeil de la personne avec des moyens d'éclairage ;
- stimuler (S14) au moins un oeil de la personne avec un stimulus lumineux afin de déclencher un réflexe lumineux pupillaire ; et
- enregistrer (S16) des images d'au moins un oeil pendant au moins une période prédéterminée suivant la stimulation d'un oeil ;
**caractérisé par** le recouvrement de façon étanche à la lumière des deux yeux de la personne et l'isolement (S10) d'un oeil par rapport à l'autre de manière étanche à la lumière en utilisant un boîtier étanche à la lumière (5) comprenant une paroi de séparation (7) pour l'isolement d'un oeil par rapport à l'autre de manière étanche à la lumière.
